# EUROPEAN PATENT APPLICATION

(11) **EP 1 582 147 A1**
(43) Date of publication of application: **05.10.2005**
(21) Application number: 03739962.3
(22) Date of filing: 19.06.2003
(51) Int. Cl.: A61B 5/05

(54) **A HUMAN BODY COMPOSITION MONITORING AND MEASURING APPARATUS OF A GLASS PLATFORM WITH FILM COATING ELECTRODES**

(30) Priority: 26.11.2002 CN 02249850
(71) Applicant: Shenzhen Healthcare Electric Technology Co., Ltd., Nan Distr. Shenzhen, Guangdong 518057 (CN); Research Institute of Tsinghua University in Shenzhen, Guangdong 518057 (CN)
(72) Inventor: LI, Yueqiu Rm. B303, Res. Institute of Tsinghua, Shen zhen, Guangdong 518057 (CN); LIU, Yan, Shenzhen Guangdong 518038 (CN)
(74) Representative: Grättinger & Partner (GbR)
(86) International application number: PCT/CN2003/000476
(87) International publication number: WO 2004/047637

(57) **Abstract**

The present invention relates to a measuring technology, in particular relates to a monitoring and measuring apparatus of a glass platform with film coating electrodes: a human body composition monitoring and measuring apparatus of a glass platform with film coating electrodes, comprises a glass measuring platform with film coating electrodes on the surface, a weighing sensor and a circuit, a microprocessor system, a body impedance and liquid measuring electrodes and a circuit, a display and a keyboard. The person to be measured stands on the glass measuring platform with feet bear, his soles contacting with the film coating electrodes. The parameters of body weight, bodyfat content or water content, etc. are measured. The apparatus is convenient to be used to measure a human body composition in daily life.

## Description

### FIELD OF THE INVENTION

The present invention relates to measurement devices in general, and more particularly to a body composition monitor by measuring bioelectrical impedance based on plating film electrodes on glass platform.

### BACKGROUND OF THE INVENTION

The method for estimating body composition is simple and credible by measuring human body bioelectrical impedance jointly using the corresponding mathematic mode. As disclosed in China patent of 'Electronic scale with human fat determining device' (ZL 00239320.4), the measuring platform is part of the scale, which is fixed with electrode on the surface. When the subject is measured with bare feet making complete contact on the electrodes, its electronic circuit could calculate the subject's body impedance. Thus the subject's body fat content is estimated by an integrated intelligent chip with other measured parameters: body weight, the input height and age of the subject. This monitor can get the two parameter of body weight and body fat content with one measurement..

As disclosed in China patent of 'Integrated monitoring apparatus for human weight, fat content and moisture content' (ZL 01235598.4), the measurement functions for main body composition parameters of weight, fat and moisture content are integrated to a whole monitor.

Measuring platform structures of these devices are all with metal electrodes fixing on plastic shell surface. This results in shortcomings of manufacturing plastic shell by plastic mould and manufacturing metal electrode by metal mould respectively.

### SUMMARY OF THE INVENTION

The present invention aims to providing a body composition monitor based on plating film electrodes on glass platform, according to which, the parameters of body weight, fat and water is determined through the connection of foot and plating electrode as the barefoot subject stands on the glass platform, and so, the monitor is convenient for body composition monitoring in our daily lives.

To reach above aim, solution of this invention is to provide a human body composition monitoring and measuring apparatus of glass platform with film coating electrodes, which comprising weighing sensor and circuit, microprocessor system, body impendence and water measuring electrodes and circuit, display, keyboard and glass platform with plating film electrodes.

The said plating film electrodes of the said human body composition monitoring and measuring apparatus comprise film electrodes and grid capacitance film electrodes; at least two film electrodes, the two electrodes are nonconductive; at least a pair of grid capacitance film electrodes, each pair contains two unconnected electrodes with equal distance, the two electrodes are nonconductive and are nonconductive to other electrodes.

For the said plating film electrodes of the said human body composition monitoring and measuring apparatus, the surface area of the said film electrodes is larger than that of the said grid capacitance film electrodes.

For the said human body composition monitoring and measuring apparatus, its plating film electrodes are connected to the port of the said body impedance and water measuring circuit. The said body impedance and water measuring circuit is connected to two ports of the said microprocessor system, one of which is the signal acquisition port of the said microprocessor system and the other is a control port for the said microprocessor system controlling the said body impedance and water measurement circuit. The signal wire of the said weighing sensor is connected with the weight signal processing circuit; the said microprocessor system is respectively connected to the said weight signal processing circuit, the said display and the said keyboard via ports.

The screen of the said display and the said keyboard are set on the top surface of the glass platform.

Compared with prior art, the advantage of this invention is that, the measurement platform, made of insulating glass base with plating film electrodes, is firm enough to bear human weight, dispensing with other metal structures. Thus cost of building plastic shell mould, medal plat electrode mould and manufacture are decreased.

### BRIEF DESCRIPTION OF THE FIGURES

Figure 1 presents schematic system structure of the invention.
Figure 2 presents schematic structure of the glass platform with film coating electrodes.
Figure 3 presents schematic theory diagrams of body impedance and partial subcutaneous organic permittivity.

### DETAILED DESCRIPTION OF THE PREFERRED EMBODIMENT

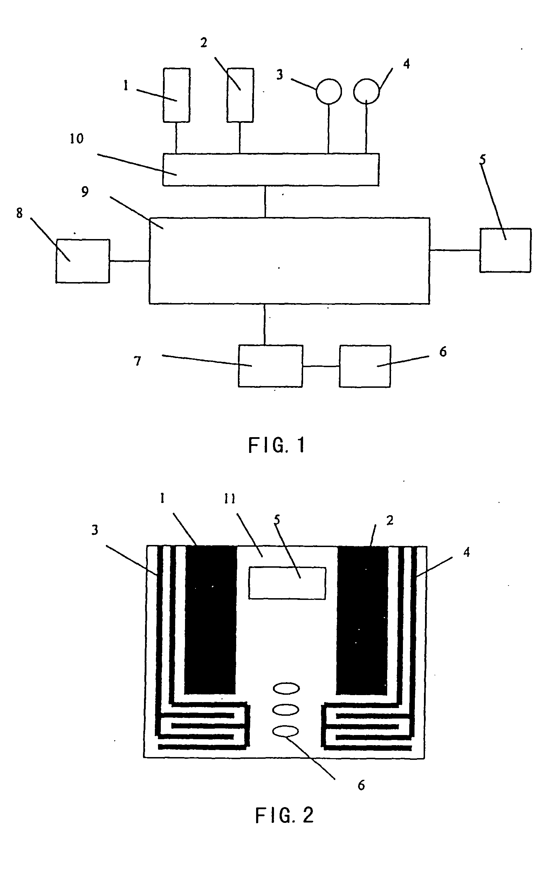

According to figure 1, on the glass platform with plating film electrodes, the film electrodes 1 and 2 for body impedance and the grid capacitance film electrodes 3 and 4 for partial subcutaneous organic permittivity measurement, are connected with the port of the body impedance and water content measurement circuit 10. Circuit 10 is connected with two ports of MCU system 9. One of these two ports is for signal acquisition of MCU systems 9 and the other control MCU system 9 for sending switch commands to the body impedance and water measurement circuit 10 to switch measuring signals between body impedance and partial subcutaneous organic permittivity. Signal wire of the weighting sensor 6 connects weighing signal processing circuit 7. Thus processed signal by circuit 7 is imported to MCU system 9 through one port of system 9. Display 5 connects ports of MCU system 9 and keyboard 8 connects one port of MCU system 9.

The said body impedance is an important parameter for measuring human body fat content.

According to figure 2, on the top surface of the insulating glass platform 11, there are two pieces of film electrodes 1 and 2, insulating to each other. Furthermore there are two pairs of grid capacitive film electrodes 3 and 4 on the top surface of the glass platform with each pair comprising two non-contact electrodes with equal distance. The two pieces of electrodes are insulating between each other and these two pairs of electrodes are also insulating from each other. Display 5 and keyboard 6 is on the glass platform and the surface area of film electrodes 1 and 2 is larger than that of the grid capacitance film electrodes.

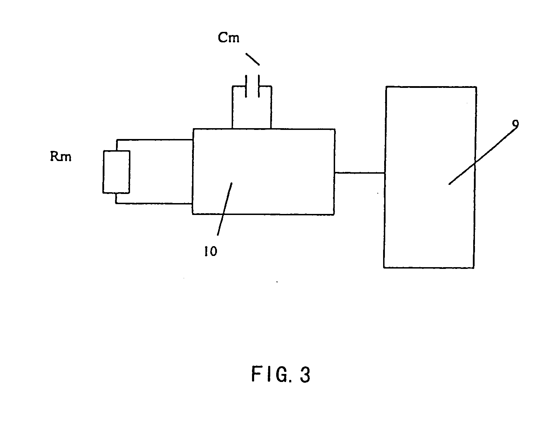

Figure 3 presents the schematic testing diagram of the invention. Body impedance and water contend measurement circuit 10 is positive feedback RC oscillation circuit. Through subject's two soles contacting electrodes 1 and 2 body impedance Rₙₐ is connected with body impedance and water content measurement circuit 10. Through the subjects' two soles contacting the capacitance film electrodes 3 and 4, body subcutaneous capacitance Cₘ is connected with body impedance and water content measurement circuit 10. Output port of body impedance and water content measurement circuit 10 is connected to MCU system 9.

Working process of the invention is as follows: when a barefoot person stands on the glass measuring board 11, his two feet separately contact the body impedance film electrodes 1 and 2 and the grid capacitance film electrodes 3 and 4 on the top of the glass platform surface. Through the output frequency signal of the body impedance and water measurement circuit 10, which is correlative with the body impedance and the partial subcutaneous permittivity, can measure the body impedance and the partial subcutaneous permittivity along with body weight from the weighting sensor 6 processed through circuit 7. After MCU system 9 operates the data including weight, body impedance, partial subcutaneous permittivity and the input information of sex, height and age, the output of body weight, body fat content and water content will be analyzed and shown on the screen of the display 5.

## Claims

1. A body composition monitor of glass platform with plating film electrodes comprising: a glass platform with film coating electrodes on its surface, which is used to measure body impedance, a weighting sensor and relative circuit. MCU system; the body impedance and water content measurement electrodes and relative circuit, display and keyboard.

2. According to claim 1, the said human body composition monitoring and measuring apparatus has characteristics as below:
The said plating film electrodes include film electrodes and grid capacitance film electrodes; at least two film electrodes with one pair consisting of two non-contact and non-conducting electrodes at equal distance. One pair is also non-conducting with the other pair.

3. According to claims 1 and 2, the said human body composition monitoring and measuring apparatus has characteristics as below:
For the said film coating electrode, the surface area of its film coating electrodes is larger than that of the grid capacitance film electrodes.

4. According to claims 1 and 2, the said human body composition monitoring and measuring apparatus has characteristics as below:
The plating film electrodes connect the ports of body impedance and water content measurement circuit which connects two ports of MCU system with one port for MCU system's signal acquisition and the other for controlling the body impedance and water content measurement circuit; signal wire from weighting sensor connects the weight signal processing circuit; MCU system connects the weight signal processing circuit, display and keyboard as well through ports.

5. According to claim 1, the said human body composition monitoring and measuring apparatus has characteristics as below:
The said display screen and keyboard are fixed on the surface of the glass platform.
